# EUROPEAN PATENT APPLICATION

(11) **EP 4 403 577 A1**
(43) Date of publication of application: **24.07.2024**
(21) Application number: 22870240.3
(22) Date of filing: 14.09.2022
(51) Int. Cl.: C07K 16/28, A61P 35/00, A61K 39/00

(54) **MODIFIED ANTI-VEGFR2 (KDR) ANTIBODY AND USE THEREOF**

(30) Priority: 15.09.2021 KR 20210122913
(71) Applicant: Pharmabcine Inc., Daejeon 34047 (KR)
(72) Inventor: NAM, Ju Ryoung, Daejeon 34047 (KR); JEONG, Jong Geun, Daejeon 34047 (KR); LEE, Weon Sup, Daejeon 34047 (KR); YOO, Jin-San, Daejeon 34047 (KR)
(74) Representative: Mewburn Ellis LLP
(86) International application number: PCT/KR2022/013664
(87) International publication number: WO 2023/043156

(57) **Abstract**

The present invention relates to a modified anti-VEGFR2 (KDR) antibody having improved properties or an antigen-binding fragment thereof, and a use thereof. Specifically, the present invention pertains to an anti-VEGFR2 (KDR) antibody, in which VH1 in the heavy chain framework region (FR) of a human antibody is substituted with VH3, VL1 in the light chain framework region (FR) of the human antibody is substituted with VK1, wherein the human antibody specifically binds to VEGFR2/KDR, and mutation is induced to increase the affinity for an antigen; or an antigen-binding fragment thereof; a composition for diagnosing or treating angiogenesis-related diseases, comprising the antibody or antigen-binding fragment thereof; a composition for preventing or treating tumor or cancer; and a composition for combined administration with a therapeutic agent other than the antibody.

## Description

### Technical Field

The present invention relates to a modified anti-VEGFR2 (KDR) antibody having improved properties or an antigen-binding fragment thereof, and a use thereof. Specifically, the present invention pertains to an anti-VEGFR2 (KDR) antibody, in which VH1 in the heavy chain framework region (FR) of a human antibody is substituted with VH3, VL1 in the light chain framework region (FR) of the human antibody is substituted with VK1, wherein the human antibody specifically binds to VEGFR2/KDR, and mutation is induced to increase the affinity for an antigen; or an antigen-binding fragment thereof; a composition for diagnosing or treating angiogenesis-related diseases, comprising the antibody or antigen-binding fragment thereof; a composition for preventing or treating tumor or cancer; and a composition for combined administration with a therapeutic agent other than the antibody.

### Related Art

VEGFR2/KDR is the primary angiogenic receptor, binds VEGF isoforms A, C, D and E, and is important for not only endothelial cell differentiation, but also the mitogenic, angiogenic, and permeability-enhancing effects of VEGF. Anti-VEGFR2 antibodies can prevent all known VEGF isofroms from binding to VEGFR2/KDR and initiating signaling. Furthermore, because tumors secrete more VEGF, while the number of receptors remains relatively constant, targeting the receptor increases the probability of completely inhibiting signaling even in the presence of very high levels of VEGF isoforms.

Angiogenesis means the formation of new blood vessels from existing blood vessels by the growth, division and migration of endothelial cells. In adults, it does not occur except in special cases such as wound healing or the female reproductive cycle. However, excessive angiogenesis has been reported in diseases such as tumor growth and metastasis, age-related macular degeneration, rheumatoid arthritis, diabetic retinopathy, psoriasis or chronic inflammation (Cameliet andJain, Nature, 407:249, 2000), and for this reason, there has been a great deal of effort to use angiogenesis inhibitors to treat diseases, especially tumors.

Factors involved in angiogenesis include vascular endothelial growth factor (VEGF), epidermal growth factor (EGF), platelet-derived growth factor (PDGF), transforming growth factor (TGFb), and fibroblast growth factor (FGF), of which endothelial growth factor is an endothelial cell-specific factor that is directly involved in differentiation and migration, and there are four different isoforms (VEGF165, VEGF121, VEGF189, VEGF206), and VEGF165 is the most abundant isoform in all human tissues except placenta (Tisher et al., J Biol Chem, 266: 11947, 1991).

Vascular endothelial growth factor (VEGF) regulates the formation of new blood vessels from the differentiation of endothelial precursors (angioblasts) in situ, is expressed in embryonic tissue (Breier et al, Development (Camb), 114:521, 1992), macrophages, and proliferating epithelial keratinocytes during wound healing (Brown et al., J. Exp. Med., 176:1375, 1992), and may be responsible for tissue edema associated with inflammation (Ferrara et al., Endocr. Rev., 13:18, 1992). In situ hybridization studies have shown that VEGF is highly expressed in a number of human tumor lines including glioblastoma multiforme, hemangioblastoma, central nervous system neoplasms, and AIDS-linked Kaposi's sarcoma (Plate et al., nature 359:845,1992; Plate et al., Cancer Res. 53:5822, 1993; Berkman et al., J. Clin. Invest. 91: 153, 1993; Nakamura et al., AIDS Weekly. 13(1), 1992). High levels of VEGF have also been observed in hypoxia-induced angiogenesis (Shweiki et al., Nature 359:843, 1992).

The biological function of VEGF is mediated through the VEGF receptor, which has a high affinity for VEGF, and is selectively expressed in endothelial cells during embryogenesis (Millauer et al., Cell, 72: 835, 1993) and during tumorigenesis. In general, the VEGF receptor (VEGFR) is a class III receptor-type tyrosine kinase characterized by having multiple, typically five or seven immunoglobulin-like loops in its amino-terminal extracellular receptor ligand-binding domain (Kaipainen et al., J. Exp. Med., 178: 2027, 1993). The other two sites include a carboxy-terminal intracellular catalytic domain and a transmembrane site that is disrupted by the insertion of a hydrophilic interkinase sequence of variable length, termed the kinase insert domain (Terman et al., Oncogene, 6: 1677, 1991). VEGFR includes the fms-like tyrosine kinase receptor (flt-1) or VEGFR-1 (Shibuya et al., OncoGene, 5:519, 1990; WO 92/14248; Terman et al, OncoGene, 6:1677, 1991), and kinase insert domain containing receptor/fetal liver kinase (KDR/flk-1), or VEGFR-2 (Matthews et al., Proc. Natl. Acad. Sci. USA, 88:9026,1991), although other receptors such as neuropilin-1 and neuropilin-2 can also bind VEGF. Another tyrosine kinase receptor, VEGFR-3 (flt-4), binds to the VEGF homologs VEGF-C and VEGF-D and plays an important role in the development of lymphatic vessels.

High levels of Flk-1 are expressed by endothelial cells infiltrating gliomas. (Plate et al., Nature 359:845, 1992). Flk-1 levels are specifically upregulated by VEGF produced by human glioblastoma (Plate et al., Cancer Res. 53:5822, 1993). The fact that Flk-1 is expressed at high levels in glioblastoma-associated endothelial cells (GAECs) indicates that receptor activity is probably induced during tumorigenesis, since Flk-1 transcripts are barely detectable in normal brain endothelial cells. This upregulation occurs only in vascular endothelial cells in close proximity to the tumor. Blocking VEGF activity with a neutralizing anti-VEGF monoclonal antibody (mAb) inhibits the growth of human tumor grafts in nude mice (Kim et al., Nature 362: 841, 1993), indicating a direct role of VEGF in tumor-associated angiogenesis.

Although VEGF ligand is upregulated in tumor cells and its receptor is upregulated in tumor-infiltrating vascular endothelial cells, expression of VEGF ligand and its receptor is low in normal cells that are not involved in angiogenesis. Therefore, these normal cells block the interaction between VEGF and its receptor, resulting in inhibition of angiogenesis and thus tumor growth.

High levels of VEGFR-2 are expressed by endothelial cells infiltrating gliomas and are specifically upregulated by VEGF produced by human glioblastomas (Plate et al., Nature, 359:845, 1992; Plate et al., Cancer Res., 53:5822, 1993). Because VEGFR-2 transcripts are rarely detected in normal brain endothelial cells, the high level of VEGFR-2 expression in glioblastoma-associated endothelial cells (GAECs) indicates that receptor activity is induced during tumorigenesis.

VEGF is expressed at high levels in many types of tumors (Plate et al., Nature, 359:8435, 1992; Dvorak et al., J. Exp. Med., 174:1275, 1991), and newly formed capillaries cluster around VEGF-producing tumor cells (Plate et al., Nature, 359:8435, 1992). VEGF expression is strongly upregulated under hypoxic conditions, such as those associated with rapidly growing tumors (Shweiki et al., Nature, 359:843, 1992). Neutralization of VEGF by antibodies such as Avastin (Ferrara et al., Nat Rev Drug Discov., 3(5):391, 2004; Avery et al., Ophthalmology, 113(3):363, 2006) is a clinically approved therapy to inhibit cancer or other angiogenic diseases such as AMD. However, resistance to VEGF blockade has been found even in combination with chemotherapy. This resistance may be associated with remodeled vasculature and increased expression of other angiogenic factors. Thus, there remains a need in the art for improved compositions and methods for inhibiting cancer and other diseases associated with angiogenesis.

Although both tyrosine kinase inhibitors (TKIs) and anti-KDRP antibodies can inhibit KDR-mediated angiogenesis, the antibody approach has advantages over TKIs. In contrast to TKIs, anti-KDR antibodies are more specific KDR targets (that is, anti-KDR antibodies do not inhibit other VEGF receptors). Because of this higher specificity, anti-KDR antibodies may limit and/or avoid off-target effects and toxicity caused by less specific TKIs (Witte et al., Cancer Metastasis Rev., Jun;17(2):155, 1998).

Unlike Avastin, which binds only one ligand (VEGF-A), anti-KDR antibodies are expected to prevent all known VEGFs from binding to VEGFR2/KDR. Anti-KDR antibodies may have a more potent inhibitory effect on tumor angiogenesis than simply blocking VEGF-A. It is possible that therapy with anti-KDR antibodies may be effective in cases of Avastin resistance.

Under this technical background, the inventors of the present invention have improved existing antibodies that specifically bind to VEGFR2/KDR by producing novel antibodies that have increased affinity for the antigen by substituting the framework region (FR) of the heavy chain variable region from VH1 to VH3 and the framework region (FR) of the light chain variable region from VL1 to VK1, and by inducing mutations in the CDR3 regions of the heavy and light chains to increase the affinity for the antigen.

### Summary of Invention

It is an object of the present invention to provide a modified antibody or an antigen-binding fragment thereof with improved binding to VEGFR2/KDR.

Another object of the present invention is to provide a nucleic acid encoding the antibody or antigen-binding fragment thereof.

Another object of the present invention is to provide a vector comprising the nucleic acid, a transgenic cell comprising the vector, and a method of making the same.

Another object of the present invention is to provide a composition for the prevention or treatment of angiogenic diseases, comprising the antibody or antigen-binding fragment thereof. Another object of the present invention is to provide a method of preventing or treating angiogenic diseases, comprising the step of administering the antibody or antigen-binding fragment thereof to an individual. Another object of the present invention is to provide a use of the antibody or antigen-binding fragment thereof in the preparation of a composition for the prevention or treatment of angiogenic diseases.

Another object of the present invention is to provide compositions for the diagnosis of angiogenic diseases comprising the antibodies or antigen-binding fragments thereof.

Another object of the present invention is to provide a composition for the prevention or treatment of a tumor or cancer, comprising the antibody or antigen-binding fragment thereof. Another object of the present invention is to provide a method of preventing or treating cancer, comprising the step of administering the antibody or antigen-binding fragment thereof to an individual. Another object of the present invention is to provide a use of the antibody or antigen-binding fragment thereof in the preparation of a composition for the prevention or treatment of cancer.

Another object of the present invention is to provide a composition for co-administration with other therapeutic agents comprising the antibody or antigen-binding fragment thereof.

To accomplish the above objectives, the present invention provides an antibody or antigen-binding fragment thereof that binds to VEGFR2/KDR, comprising:
a heavy chain CDR1 comprising a sequence selected from the group consisting of SEQ ID Nos: 1, 7, 13, 19 and 25,
a heavy chain CDR2 comprising a sequence selected from the group consisting of SEQ ID Nos: 2, 8, 14, 20 and 26; and
a heavy chain variable region comprising a heavy chain CDR3 comprising a sequence selected from the group consisting of SEQ ID Nos: 3, 9, 15, 21, 27, 51, 52 and 53; and
a light chain CDR1 comprising a sequence selected from the group consisting of SEQ ID Nos: 4, 10, 16, 22 and 28,
a light chain CDR2 comprising a sequence selected from the group consisting of SEQ ID Nos: 5, 11, 17, 23 and 29; and
a light chain variable region comprising a light chain CDR3 comprising a sequence selected from the group consisting of SEQ ID Nos: 6, 12, 18, 24 and 30.

The present invention also provides an antibody or antigen-binding fragment thereof that binds to VEGFR2/KDR, comprising a heavy chain variable region comprising a sequence having at least 80% identity to a sequence selected from the group consisting of SEQ ID Nos: 32, 36, 40, 44 and 48.

The present invention also provides an antibody or antigen-binding fragment thereof that binds to VEGFR2/KDR, comprising a light chain variable region comprising a sequence having at least 80% identity to a sequence selected from the group consisting of SEQ ID Nos: 34, 38, 42, 46 and 50.

The present invention also provides an antibody or antigen-binding fragment thereof that binds to VEGFR2/KDR, comprising a heavy chain variable region comprising a sequence having at least 80% identity to a sequence selected from the group consisting of SEQ ID Nos: 32, 36, 40, 44 and 48, and a light chain variable region comprising a sequence having at least 80% identity to a sequence selected from the group consisting of SEQ ID Nos: 34, 38, 42, 46 and 50.

The present invention also provides a nucleic acid encoding the antibody or antigen-binding fragment thereof.

The present invention also provides a vector comprising the nucleic acid.

The present invention also provides a transgenic cell comprising the vector.

The present invention also provides a method of producing the antibody or antigen-binding fragment thereof, comprising the steps of (a) culturing the cells; and (b) recovering the antibody or antigen-binding fragment thereof from the cultured cells.

The present invention also provides a composition for the prevention or treatment of angiogenic diseases, comprising the antibody or antigen-binding fragment thereof.

The present invention also provides a composition for the diagnosis of angiogenic diseases, comprising the antibody or antigen-binding fragment thereof.

The present invention also provides a composition for the prevention or treatment of a tumor or cancer comprising the antibody or antigen-binding fragment thereof.

The present invention also provides a composition for co-administration with other therapeutic agents comprising the antibody or antigen-binding fragment thereof.

### Brief Description of Drawing

FIG. 1 shows sequences to which the heavy chain CDR sequence of 6A6 is transplanted.
FIG. 2 shows sequences to which the light chain CDR sequence of 6A6 is transplanted.
FIG. 3 shows the results of an ELISA evaluating the binding of transiently expressed and purified anti-Ang2 antibodies to human and mouse Ang2 and Ang1.
FIG. 4 shows results demonstrating the ability of neutralizing Ang2/Tie2 binding of selected human and mouse with anti-Ang2 antibodies.
FIG. 5 shows results demonstrating the ability of neutralizing the binding of Ang2/integrin of selected anti-Ang2 antibodies.
FIG. 6 shows that selected anti-Ang2 antibodies can inhibit Ang2/Tie2 signaling.
FIG. 7 shows the results of confirming the purity of the purification steps by expressing selected anti-Ang2 scFv antibodies in E. coli.
FIG. 8 shows the results of an ELISA evaluating the binding of an anti-Ang2 scFv antibody expressed in E. coli to human Ang2 protein.
FIG. 9 shows the ability of inhibiting angiogenesis of anti-Ang2 scFv antibody expressed in E. coli in CNV animals.

### Detailed Description of the invention and Preferred Embodiments

Unless otherwise defined, all technical and scientific terms used herein have the same meaning as commonly understood by a person skilled in the art. In general, the nomenclature used herein is well known and in common use in the art.

The present invention relates to a modified antibody of a fully humanized antibody that neutralizes the vascular endothelial growth factor receptor. In particular, it relates to a modified antibody of a human monoclonal antibody that neutralizes the vascular endothelial growth factor receptor (Korean Patent No. 10-0883430) and a use thereof.

The inventors of the present invention have developed the only antibody that is invented from a fully human antibody library, targets VEGFR-2/KDR, and is also reactive against mouse or rat-derived flk-1 (VEGFR-2 homolog). Although this antibody has proven to be excellent in clinical trials, the antibody protein has a rather low binding to the antigen and a short in vivo half-life. By improving these properties, it is expected to increase its efficacy as an immuno-oncology drug and improve its efficacy in angiogenesis-related diseases.

To improve an existing antibody that specifically binds to VEGFR2/KDR, a novel antibody with increased affinity for the antigen was prepared by substituting the framework region (FR) of the heavy chain variable region from VH1 to VH3 and the framework region (FR) of the heavy chain variable region from VL1 to VK1, and by inducing mutations in the CDR3 regions of the heavy and light chains. As a result, the inventors of the present invention developed an antibody with higher binding and better intracellular efficacy than existing antibodies that specifically bind to VEGFR2/KDR, confirming that the antibody may serve as a desired immuno-oncology drug or a therapeutic agent for angiogenesis-related diseases.

The modified antibody was screened as follows. First, the CDR region sequence and framework (FR) sequence of 6A6 were identified, and using the CDR grafting technique, the heavy chain CDR sequence was grafted into the human germline VH3 sequence, and the light chain CDR sequence was grafted into the human germline VK1.

CDR grafting is typically a method in which the DNA encoding the variable region sequence of a mouse monoclonal antibody is isolated, the CDR sequence is obtained by gene cloning, and the CDR sequence is then grafted into a suitable human antibody sequence *in silico* (Hou S, et al. Humanization of an anti-CD34 monoclonal antibody by complementarity-determining region grafting based on computer-assisted molecular modelling. J Biochem. 2008;144(1):115-20; and Kashmiri SV, De Pascalis R, Gonzales NR, Schlom J. SDR grafting--a new approach to antibody humanization. Methods. 2005;36(1):25-34). Utilizing the above method, the framework of 6A6 with human germline VH1/VL1 was substituted with human germline VH3/VK1.

To improve the affinity of the substituted antibody, libraries inducing mutations in the CDR3 base sequences of the light and heavy chains were constructed, and the antibody was screened using recombinant KDR D1 to D3-Fc fusion proteins. Antibody screening was performed using phage display.

Based on this, the present invention provides, in one aspect, an antibody or antigen-binding fragment thereof that binds to VEGFR2/KDR, comprising:
a heavy chain CDR1 comprising a sequence selected from the group consisting of SEQ ID Nos: 1, 7, 13, 19 and 25,
a heavy chain CDR2 comprising a sequence selected from the group consisting of SEQ ID Nos: 2, 8, 14, 20 and 26; and
a heavy chain variable region comprising a heavy chain CDR3 comprising a sequence selected from the group consisting of SEQ ID Nos: 3, 9, 15, 21, 27, 51, 52 and 53; and
a light chain CDR1 comprising a sequence selected from the group consisting of SEQ ID Nos: 4, 10, 16, 22 and 28,
a light chain CDR2 comprising a sequence selected from the group consisting of SEQ ID Nos: 5, 11, 17, 23 and 29; and
a light chain variable region comprising a light chain CDR3 comprising a sequence selected from the group consisting of SEQ ID Nos: 6, 12, 18, 24 and 30.

As used herein, the term "antibody" refers to an anti-VEGFR2/KDR antibody that specifically binds to VEGFR2/KDR. The scope of the present invention includes not only complete antibody forms that specifically bind to VEGFR2/KDR, but also antigen-binding fragments of the antibody molecules.

A complete antibody is a structure with two full-length light chains and two full-length heavy chains, each of which light chains is linked to the heavy chain by a disulfide bond. The heavy chain invariant region is of the gamma (γ), mu (µ), alpha (α), delta (δ), and epsilon (ε) types with subclasses gamma1 (γ1), gamma2 (γ2), gamma3 (γ3), gamma4 (γ4), alpha1 (α1), and alpha2 (α2). The light chain invariant region has kappa (κ) and lambda (λ) types.

Antigen-binding fragment or antibody fragment of an antibody refers to a fragment that possesses antigen-binding function, including Fab, F(ab'), F(ab')2, and Fv. Among the antibody fragments, Fab is a structure with variable regions of light and heavy chains, invariant regions of light chain and the first invariant region (CH1) of heavy chain, and has one antigen-binding site. Fab' differs from Fab in that it has a hinge region containing one or more cysteine residues at the C-terminus of the heavy chain CH1 domain. F(ab')2 antibody is generated by disulfide bonding of cysteine residues in the hinge region of Fab'. Fv is a minimal antibody fragment that has only a heavy chain variable region and a light chain variable region. In a two-chain Fv (two-chain Fv), the heavy and light chain variable regions are connected by a non-covalent linkage, and in a single-chain Fv (scFv), the heavy and light chain variable regions are connected by a covalent linkage usually via a peptide linker, or directly connected at the C-terminus, which can form a dimer-like structure like a double-chain Fv. This antibody fragment can be obtained using proteolytic enzymes (e.g., restriction digestion of the whole antibody with papain yields Fab and digestion with pepsin yields F(ab')2 fragments), or they can be produced by genetic recombination techniques.

In one embodiment, the antibody according to the present invention is in the form of an Fv (e.g., scFv), or is in the form of a complete antibody. Further, the heavy chain invariant region may be selected from any one of the following isotypes: gamma (γ), mu (µ), alpha (α), delta (δ) and epsilon (ε). For example, the invariant region is gamma 1 (lgG1), gamma 3 (IgG3), or gamma 4 (IgG4). The light chain invariant region may be the kappa or lambda isotype.

As used herein, the term "heavy chain" refers to both of full-length heavy chain and fragment thereof comprising a variable region domain VH and three invariant domains CH1, CH2, and CH3 comprising an amino acid sequence having sufficient variable region sequence to confer specificity to an antigen. Furthermore, as used herein, the term "light chain" refers to both of full-length light chain and fragment thereof comprising a variable region domain VL and an invariant region domain CL comprising an amino acid sequence having sufficient variable region sequence to confer specificity to the antigen.

The antibodies of the present invention include, but are not limited to, monoclonal antibodies, multispecific antibodies, human antibodies, humanized antibodies, chimeric antibodies, short-chain Fvs (scFVs), short-chain antibodies, Fab fragments, F(ab') fragments, disulfide-bound Fvs (sdFVs), and anti-idiotype (anti-ld) antibodies, or epitope-binding fragments of the above antibodies.

The monoclonal antibody refers to an antibody obtained from a substantially homogeneous population of antibodies, i.e., the individual antibodies in the population are identical except for possible naturally occurring mutations that may be present in trace amounts. Monoclonal antibodies are highly specific, meaning that they are directed against a single antigenic site. In contrast to conventional (polyclonal) antibody preparations, which typically contain different antibodies directed against different determinants (epitopes), each monoclonal antibody is directed against a single determinant on the antigen.

An "epitope" is a protein determinant to which an antibody can specifically bind. Epitopes typically consist of a group of chemically active surface molecules, such as amino acids or sugar side chains, which typically have specific charge characteristics as well as specific three-dimensional structural features. Steric and non-steric epitopes are distinguished in that binding to the former is lost in the presence of a denaturing solvent, but not to the latter.

Non-human (e.g., murine) antibodies in their "humanized" form are chimeric antibodies containing a minimal sequence derived from a non-human immunoglobulin. In most cases, a humanized antibody is a human immunoglobulin (recipient antibody) in which residues from the hypervariable region of the recipient are replaced with residues from the hypervariable region of a non-human species (donor antibody), e.g., mouse, rat, rabbit, or non-human primate, that possesses the specificity, affinity, and ability to target the recipient.

The "human antibody" means a molecule derived from a human immunoglobulin, wherein the entire amino acid sequence consisting of the antibody including complementary crystal regions and structural regions is composed of human immunoglobulin.

While a portion of the heavy and/or light chain is identical or homologous to a corresponding sequence in an antibody derived from a particular species or belonging to a particular antibody class or subclass, the remaining chain(s) may include a "chimeric" antibody (immunoglobulin) which is identical or homologous to a corresponding sequence in an antibody derived from another species or belonging to another antibody class or subclass, or a fragment of the antibody exhibiting the desired biological activity.

As used herein, "antibody variable region" refers to the light and heavy chain portions of an antibody molecule containing the amino acid sequences of the complementarity determining region (CDR; i.e., CDR1, CDR2, and CDR3), and the backbone region (FR). VH refers to the variable region of the heavy chain. VL refers to the variable region of the light chain.

"Complementarity determining region" (CDR; i.e., CDR1, CDR2, and CDR3) refers to an amino acid residue in an antibody's variable region that is required for antigen binding. Each variable region typically has three CDR regions, identified as CDR1, CDR2, and CDR3. The present invention includes a heavy chain variable region comprising a heavy chain CDR3 of sequence ID No: 1 and a light chain variable region comprising a light chain CDR3 of sequence ID No: 2.

In the present invention, the antibody or antigen-binding fragment thereof that binds to VEGFR2/KDR may comprise:
a heavy chain variable region comprising a heavy chain CDR1 of SEQ ID NO: 1, a heavy chain CDR2 of SEQ ID NO: 2, and a heavy chain CDR3 of SEQ ID NO: 3; and a light chain variable region comprising a light chain CDR1 of SEQ ID NO: 4, a light chain CDR2 of SEQ ID NO: 5, and a light chain CDR3 of SEQ ID NO: 6,
a heavy chain variable region comprising a heavy chain CDR1 of SEQ ID NO: 7, a heavy chain CDR2 of SEQ ID NO: 8, and a heavy chain CDR3 of SEQ ID NO: 9; and a light chain variable region comprising a light chain CDR1 of SEQ ID NO: 10, a light chain CDR2 of SEQ ID NO: 11, and a light chain CDR3 of SEQ ID NO: 12,
a heavy chain variable region comprising a heavy chain CDR1 of SEQ ID NO: 13, a heavy chain CDR2 of SEQ ID NO: 14, and a heavy chain CDR3 of SEQ ID NO: 15; and a light chain variable region comprising a light chain CDR1 of SEQ ID NO: 16, a light chain CDR2 of SEQ ID NO: 17, and a light chain CDR3 of SEQ ID NO: 18,
a heavy chain variable region comprising a heavy chain CDR1 of SEQ ID NO: 19, a heavy chain CDR2 of SEQ ID NO: 20, and a heavy chain CDR3 of SEQ ID NO: 21; and a light chain variable region comprising a light chain CDR1 of SEQ ID NO: 22, a light chain CDR2 of SEQ ID NO: 23, and a light chain CDR3 of SEQ ID NO: 24,
a heavy chain variable region comprising a heavy chain CDR1 of SEQ ID NO: 25, a heavy chain CDR2 of SEQ ID NO: 26, and a heavy chain CDR3 of SEQ ID NO: 27; and a light chain variable region comprising a light chain CDR1 of SEQ ID NO: 28, a light chain CDR2 of SEQ ID NO: 29, and a light chain CDR3 of SEQ ID NO: 30,
a heavy chain variable region comprising a heavy chain CDR1 of SEQ ID NO: 13, a heavy chain CDR2 of SEQ ID NO: 14, and a heavy chain CDR3 of SEQ ID NO: 51; and a light chain variable region comprising a light chain CDR1 of SEQ ID NO: 16, a light chain CDR2 of SEQ ID NO: 17, and a light chain CDR3 of SEQ ID NO: 18,
a heavy chain variable region comprising a heavy chain CDR1 of SEQ ID NO: 13, a heavy chain CDR2 of SEQ ID NO: 14, and a heavy chain CDR3 of SEQ ID NO: 52; and a light chain variable region comprising a light chain CDR1 of SEQ ID NO: 16, a light chain CDR2 of SEQ ID NO: 17, and a light chain CDR3 of SEQ ID NO: 18; or
a heavy chain variable region comprising a heavy chain CDR1 of SEQ ID NO: 13, a heavy chain CDR2 of SEQ ID NO: 14, and a heavy chain CDR3 of SEQ ID NO: 53; a light chain variable region comprising a light chain CDR1 of SEQ ID NO: 16, a light chain CDR2 of SEQ ID NO: 17, and a light chain CDR3 of SEQ ID NO: 18.

"Skeletal regions" (FRs) are variable region residues other than CDR residues. Each variable region typically has four FRs, identified as FR1, FR2, FR3, and FR4.

A "Fv" fragment is an antibody fragment that contains complete antibody recognition and binding sites. These regions consist of a dimer in which one heavy chain variable region and one light chain variable region are tightly and substantially covalently linked with, e.g., scFv.

A "Fab" fragment contains variable and invariant domains of the light chain, and variable and first invariant domains (CH1) of the heavy chain. F(ab')2 antibody fragment typically contains a pair of Fab fragments that are covalently linked near their carboxy terminus by a hinge cysteine between them.

A "single-chain Fv" or "scFv" antibody fragment contains the VH and VL domains of the antibody, which are present within a single polypeptide chain. The Fv polypeptide may additionally contain a polypeptide linker between the VH and VL domains that allow the scFv to form the desired structure for antigen binding.

A VEGFR2/KDR antibody can contain a single or double chain. Functionally, the binding affinity of a VEGFR2/KDR antibody is in the range of 10⁻⁵ M to 10⁻¹² M. For example, a VEGFR2/KDR antibody has a binding affinity of 10⁻⁶ M to 10⁻¹² M, 10⁻⁷ M to 10⁻¹² M, 10⁻⁸ M to 10⁻¹² M, 10⁻⁹ M to 10⁻¹² M, 10⁻⁵M to 10⁻¹¹ M, 10⁻⁶ M to 10⁻¹¹ M, 10⁻⁷ M to 10⁻¹¹ M, 10⁻⁸ M to 10⁻¹¹ M, 10⁻⁹ M to 10⁻¹¹ M, 10⁻¹⁰ M to 10⁻¹¹ M, 10⁻⁵ M to 10⁻¹⁰ M, 10⁻⁶ M to 10⁻¹⁰ M, 10⁻⁷ M to 10⁻¹⁰ M, 10⁻⁸ M to 10⁻¹⁰ M, 10⁻⁹ M to 10⁻¹⁰ M, 10⁻⁵ M to 10⁻⁹ M, 10⁻⁶ M to 10 M, 10⁻⁹⁻⁷ M to 10⁻⁹ M, 10⁻⁸ M to 10⁻⁹ M, 10⁻⁵ M to 10⁻⁸ M, 10⁻⁶ M to 10⁻⁸ M, 10⁻⁷ M to 10⁻⁸ M, 10⁻⁵ M to 10⁻⁷ M, 10⁻⁶ M to 10⁻⁷ M, or 10⁻⁵ M to 10⁻⁶ M.

The present invention relates to an antibody or antigen-binding fragment thereof that binds to VEGFR2/KDR, comprising a heavy chain variable region comprising a sequence having at least 80% identity to a sequence selected from the group consisting of SEQ ID Nos: 32, 36, 40, 44 and 48.

The present invention also relates to an antibody or antigen-binding fragment thereof that binds to VEGFR2/KDR, comprising a light chain variable region comprising a sequence having at least 80% identity to a selected sequence selected from the group consisting of SEQ ID Nos: 34, 38, 42, 46 and 50.

The present invention also relates to an antibody or antigen-binding fragment thereof that binds to VEGFR2/KDR, comprising a heavy chain variable region comprising a sequence having at least 80% identity to a sequence selected from the group consisting of SEQ ID Nos: 32, 36, 40, 44 and 48, and a light chain variable region comprising a sequence having at least 80% identity to a sequence selected from the group consisting of SEQ ID Nos: 34, 38, 42, 46 and 50.

The identity may be 80%, 81%, 82%, 83%, 84%, 85%, 86%, 87%, 88%, 89%, 90%, 91%, 92%, 93%, 94%, 95%, 96%, 97%, 98%, 99% or more relative to the entire sequence of the claimed SEQ ID numbers.

The antibody or antigen-binding fragment thereof that binds to the VEGFR2/KDR may comprise a heavy chain variable region selected from the group consisting of SEQ ID Nos: 32, 36, 40, 44 and 48. Furthermore, the antibody or antigen-binding fragment thereof that binds to VEGFR2/KDR may comprise a light chain variable region selected from the group consisting of SEQ ID Nos. 34, 38, 42, 46 and 50.

In a specific embodiment according to the present invention, the antibody or antigen-binding fragment thereof that binds to VEGFR2/KDR may include:
a heavy chain variable region of SEQ ID NO: 32 and a light chain variable region of SEQ ID NO: 34;
a heavy chain variable region of SEQ ID NO: 36 and a light chain variable region of SEQ ID NO: 38;
a heavy chain variable region of SEQ ID NO: 40 and a light chain variable region of SEQ ID NO: 42;
a heavy chain variable region of SEQ ID NO: 44 and a light chain variable region of SEQ ID NO: 46; or
a heavy chain variable region of SEQ ID NO: 48 and a light chain variable region of SEQ ID NO: 50.

A "phage display" is a technique for displaying a variant polypeptide as a fusion protein with at least part of the envelope protein on the surface of a phage, e.g., a fibrous phage particle. The utility of phage display lies in the fact that it can be used against large libraries of randomized protein variants, allowing rapid and efficient sorting of sequences that bind with high affinity to target antigens. Displaying peptide and protein libraries on phage has been used to screen millions of polypeptides to identify polypeptides with specific binding properties.

Phage display technology has provided a powerful tool for generating and screening novel proteins that bind specific ligands (e.g., antigens). By using phage display technology, large libraries of protein variants can be generated and sequences that bind with high affinity to target antigens can be rapidly sorted. The nucleic acid encoding the variant polypeptide is fused with a nucleic acid sequence encoding a viral envelope protein, such as a gene III protein or a gene VIII protein. A monovalent phage display system has been developed in which a nucleic acid sequence encoding a protein or polypeptide is fused with a nucleic acid sequence encoding a portion of a gene III protein. In a monovalent phage display system, the gene fusion is expressed at low levels and the wild-type gene III protein is also expressed to maintain particle infectivity.

Demonstrating the expression of a peptide on the surface of a fibrous phage and the expression of a functional antibody fragment in the periplasm of E. coli is critical to the development of an antibody phage display library. Libraries of antibodies or antigen-binding polypeptides have been prepared in a number of ways, for example, by altering a single gene by inserting random DNA sequences or by cloning a family of related genes. The library can be screened for expression of antibody or antigen-binding protein with the desired properties.

Phage display technology has several advantages over conventional hybridoma and recombinant methods for producing an antibody with desired features. This technology allows the generation of large antibody libraries with diverse sequences in a short time without the use of animals. The production of hybridomas or humanized antibodies can require months of production time. In addition, because no immunity is required, phage antibody libraries can generate antibodies against toxic or low-antigenic antigens. Phage antibody libraries can also be used to generate and identify novel therapeutic antibodies.

Phage display libraries can be used to generate human antibodies from immunized or non-immunized human, germline sequences, or unsensitized B cell Ig repertoires. Various lymphoid tissues can be used to generate unsensitized or non-immunized antigen binding libraries.

The ability to identify and isolate high-affinity antibodies from phage display libraries is important for the isolation of novel antibodies for therapeutic use. Isolation of high-affinity antibodies from libraries can depend on the size of the library, the efficiency of production in bacterial cells, and the diversity of the library. The size of the library is reduced by inefficient production due to improper folding of the antibody or antigen-binding protein and the presence of stop codons. Expression in bacterial cells can be inhibited if the antibody or antigen-binding domain is not folded properly. Expression can be improved by alternatively mutating residues on the surface of a variable/invariable interface or in selected CDR residues. The sequence of the backbone region is one factor for providing proper folding when generating antibody phage libraries in bacterial cells.

In high-affinity antibody isolation, it is important to generate diverse libraries of antibodies or antigen-binding proteins. The CDR3 region has found to be the region in which they often participate in antigen binding. CDR3 regions on heavy chains considerably vary in size, sequence, and structural stereomorphology, which can be used to generate diverse libraries.

Diversity can also be generated by randomizing the CDR regions of the variable heavy and light chains using all 20 amino acids at each position. Using all 20 amino acids can generate variant antibody sequences with greater diversity and increase the chance of identifying novel antibodies.

The antibody or antibody fragment of the present invention can include not only the sequences of the anti-VEGFR2/KDR antibody of the present invention described herein, but also biological equivalents thereof, within the range that it is capable of specifically recognizing VEGFR2/KDR. For example, additional modifications can be made to the amino acid sequence of the antibody to further improve the binding affinity and/or other biological properties of the antibody. Such modifications include, for example, deletions, insertions, and/or substitutions of amino acid sequence residues of the antibody. These amino acid modifications are made based on the relative similarity of the amino acid side chain substituents, e.g., hydrophobicity, hydrophilicity, charge, size, etc. By analyzing the size, shape, and type of amino acid side chain substituents, it can be seen that arginine, lysine, and histidine are all positively charged residues; alanine, glycine, and serine have similar sizes; and phenylalanine, tryptophan, and tyrosine have similar shapes. Therefore, based on these considerations, arginine, lysine, and histidine; alanine, glycine, and serine; and phenylalanine, tryptophan, and tyrosine are biologically functional equivalents.

When considering the variants with biologically equivalent activity described above, the antibody of the present invention or the nucleic acid molecule encoding the same are also interpreted to include sequences that exhibit substantial identity to the sequences set forth in the SEQ ID numbers. The substantial identity means a sequence that exhibits at least 90% identity, most preferably at least 95% identity, at least 96% identity, at least 97% identity, at least 98% identity, or at least 99% identity, when the sequence of the present invention is aligned with any other sequence to correspond as much as possible, and the aligned sequence is analyzed using algorithms commonly known in the art. Alignment methods for sequence comparison are known in the art. The NCBI Basic Local Alignment Search Tool (BLAST) is accessible at NCBI and elsewhere, and is available on the Internet in conjunction with sequence analysis programs such as blastp, blasm, blastx, tblastn or and tblastx. BLSAT can be accessed at www.ncbi.nlm.nih.gov/BLAST/. Instructions for comparing sequence identity using this program are available at www.ncbi.nlm.nih.gov/BLAST/blast_ help.html.

Based on this, an antibody or an antigen-binding fragment thereof of the present invention may have 90%, 91%, 92%, 93%, 94%, 95%, 96%, 97%, 98%, 99% or greater identity, compared to part or all of sequences set forth in the specification. Such identity can be determined by sequence comparison and/or alignment by methods known in the art. For example, sequence comparison algorithms (e.g., BLAST or BLAST 2.0), manual alignment, and visual inspection can be used to determine the percent sequence identity of the nucleic acid or protein of the present invention.

In another aspect, the present invention relates to a nucleic acid encoding the antibody or an antigen-binding fragment thereof.

The nucleic acid may be selected from the group consisting of SEQ ID Nos: 31, 35, 39, 43 and 47 encoding a heavy chain variable region. The nucleic acid may be selected from the group consisting of SEQ ID Nos: 33, 37, 41, 45 and 49 encoding a light chain variable region.

The antibody or antigen-binding fragment thereof can be produced recombinantly by isolating the nucleic acid encoding the antibody or antigen-binding fragment thereof of the present invention. The nucleic acid is isolated, and inserted into a replicable vector for further cloning (amplification of DNA) or further expression. Based on this, the present invention in another aspect relates to a vector comprising the nucleic acid.

The term "nucleic acid" has a broad meaning to include DNA (gDNA and cDNA) and RNA molecule, and the nucleotide, which is the basic constituent unit of a nucleic acid, includes not only a naturally occurring nucleotide but also an analog with sugar or base site modifications. The sequence of the nucleic acid encoding the heavy and light chain variable regions of the present invention may be modified. The modifications include additions, deletions, or non-conservative or conservative substitutions of nucleotides.

The DNA encoding the antibody is readily isolated or synthesized using conventional procedures (e.g., by using oligonucleotide probes that can specifically bind to DNA encoding the heavy and light chains of the antibody). Many vectors are available. Vector components typically include, but are not limited to, one or more of the following: a signal sequence, a replication site, one or more marker genes, an enhancer element, a promoter, and a transcription termination sequence.

As used herein, the term "vector" includes a plasmid vector; a cosmid vector; or a viral vector such as a bacteriophage vector, an adenoviral vector, a retroviral vector or an adeno-associated viral vector as a means for expressing a target gene in a host cell. In the vector, the nucleic acid encoding the antibody is operatively linked to a promoter.

The term "operationally linked" means a functional association between a nucleic acid expression regulatory sequence (e.g., a promoter, signal sequence, or array of transcriptional regulator binding sites) and another nucleic acid sequence, whereby the regulatory sequence regulates the transcription and/or decoding of the other nucleic acid sequence.

In the case of prokaryotic cell as a host, it is common to include a strong promoter capable of driving transcription (e.g., a tac promoter, lac promoter, lacUV5 promoter, Ipp promoter, pLλ promoter, pRλ promoter, rac5 promoter, amp promoter, recA promoter, SP6 promoter, trp promoter, and T7 promoter), a rhizosome binding site for initiation of degradation, and a transcription/degradation termination sequence. In addition, for example, in the case of eukaryotic cell as a host, a promoter derived from the genome of a mammalian cell (e.g., metallothionein promoter, β-actin promoter, human hemoglobin promoter, and human muscle creatine promoter) or a promoter derived from a mammalian virus (e.g., adenovirus late promoter, vaccinia virus 7.5K promoter, SV40 promoter, cytomegalovirus (CMV) promoter, tk promoter of HSV, mouse mammary tumor virus (MMTV) promoter, LTR promoter of HIV, Moloney virus promoter, Epstein-Barr virus (EBV) promoter, and Roos Sakoma virus (RSV) promoter) may be utilized and typically have a polyadenylation sequence as a transcription termination sequence.

In some cases, the vector may be fused with other sequences to facilitate purification of antibodies expressed therefrom. Examples of fused sequences include glutathione S-transferase (Pharmacia, USA), maltose binding protein (NEB, USA), FLAG (IBI, USA), and 6x His (hexahistidine; Quiagen, USA).

The vector comprises as a selective marker an antibiotic resistance gene conventionally used in the art, for example, a resistance gene for ampicillin, gentamicin, carbenicillin, chloramphenicol, streptomycin, kanamycin, geneticin, neomycin, and tetracycline.

In another aspect, the present invention relates to a cell transformed with the above-mentioned vector. The cell used to generate the antibody of the present invention can be, but is not limited to, a prokaryotic, yeast or higher eukaryotic cell.

Prokaryotic host cells such as Escherichia coli, strains of the genus Bacillus such as Bacillus subtilis or Bacillus churringensis, Streptomyces, Pseudomonas (e.g, Pseudomonas putida), Proteus mirabilis, or Staphylococcus (e.g., Staphylococcus carnosus) can be used.

However, animal cells are of most interest, and examples of useful host cell lines may include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S or HT1080.

In another aspect, the present invention relates to a method of producing an antibody or antigen-binding fragment thereof, comprising the steps of (a) culturing the cells; and (b) recovering the antibody or antigen-binding fragment thereof from the cultured cells.

The cells can be cultured in a variety of media. Any of the commercially available media can be used as culture media without limitation. All other necessary supplements known to those skilled in the art may also be included in suitable concentrations. Culture conditions, e.g., temperature, pH, etc., are already used with selected host cells for expression, and it would be apparent to those skilled in the art.

Recovery of the antibody or antigen-binding fragment thereof can be accomplished by removing impurities, for example by centrifugation or ultrafiltration, and purifying the resulting product, for example by affinity chromatography. Additional other purification techniques may be used, for example, anion or cation exchange chromatography, hydrophobic interaction chromatography, hydroxylapatite chromatography, and the like.

The antibody may be an IgG or a fragment comprising a variable region, e.g., ScFv or Fab. Furthermore, the variable region of the heavy chain can be IgG1, IgG2, IgG3 or IgG4.

In another aspect, the present invention relates to a composition for the prevention or treatment of angiogenic diseases, comprising the antibody or antigen-binding fragment thereof as an active ingredient.

The "angiogenesis" means the formation or growth of new blood vessels from previously existing blood vessels, and "angiogenesis-related disease" means a disease associated with the occurrence or progression of angiogenesis. A disease may be included within the scope of an angiogenesis-related disease without limitation if it can be treated with the antibody. Examples of angiogenesis-related diseases include, but are not limited to, cancer, metastasis, diabetic retinopathy, retinopathy of prematurity, corneal graft rejection, macular degeneration, neovascular glaucoma, erythrosis, proliferative retinopathy, psoriasis, hemophilic arthritis, capillary formation in atherosclerotic plaques, keloid, wound granulation, vascular adhesion, rheumatoid arthritis, osteoarthritis, autoimmune diseases, Crohn's disease, restenosis, atherosclerosis, intestinal adhesions, cat scratch disease, ulcers, liver cirrhosis, nephritis, diabetic nephropathy, diabetic foot ulcers, chronic kidney failure, diabetes mellitus, inflammatory diseases, idiopathic pulmonary fibrosis, sepsis, acute respiratory distress syndrome, and neurodegenerative diseases.

Furthermore, the cancer may be selected from the group consisting of, but is not limited to, esophageal cancer, stomach cancer, large intestine cancer, rectal cancer, oral cancer, pharynx cancer, larynx cancer, lung cancer, colon cancer, breast cancer, uterine cervical cancer, endometrial cancer, ovarian cancer, prostate cancer, testis cancer, bladder cancer, renal cancer, liver cancer, pancreatic cancer, bone cancer, connective tissue cancer, skin cancer, brain cancer, thyroid cancer, leukemia, Hodgkin's lymphoma, lymphoma, and multiple myeloid blood cancer.

As used herein, the term "prevention or prophylaxis" refers to any action that results in inhibiting or delaying the onset of a condition of concern by administering the antibody or composition of the present invention. The term "treatment or therapy" as used herein refers to any action that improves or reduces symptoms of a condition of concern by administering the antibody or composition of the present invention.

The present invention may be, for example, a pharmaceutical composition for the prevention or treatment of a tumor or cancer, comprising (a) a pharmaceutically effective amount of an antibody to VEGFR2/KDR or an antigen-binding fragment thereof according to the present invention; and (b) a pharmaceutically acceptable carrier. The present invention may also be a method of preventing or treating a tumor or cancer, comprising the step of administering the antibody or antigen-binding fragment thereof to a patient with a tumor or cancer. The present invention may further relate to use of the antibody or antigen-binding fragment thereof for interfering with the mechanism of VEGFR2/KDR and thereby preventing or treating a tumor or cancer.

A tumor or cancer that is a disease to which the composition is applied typically includes a tumor or cancer that overexpresses VEGF or VEGFR2/KDR, and a tumor or cancer that does not overexpress VEGF or VEGFR2/KDR. Non-limiting examples of tumors or cancers desirable for treatment include melanoma (e.g., metastatic malignant melanoma), kidney cancer (e.g., clear cell carcinoma), prostate cancer (e.g., hormone refractory prostate adenocarcinoma), pancreatic adenocarcinoma, breast cancer, colon cancer, lung cancer (e.g., non-small cell lung cancer), esophageal cancer, head and neck squamous cell carcinoma, liver cancer, ovarian cancer, cervical cancer, thyroid cancer, glioblastoma, glioma, leukemia, lymphoma, and other neoplastic carcinomas. Further, the present invention encompasses refractory or recurrent cancers that can be treated using the antibodies of the present invention.

The present invention provides a method of treating angiogenic diseases, comprising, for example, administering a therapeutically effective amount of the anti-VEGFR2/KDR antibody or antigen-binding fragment thereof to a patient in need of inhibition of angiogenesis. The treatment of the angiogenic diseases may further comprise the step of identifying a patient in need of angiogenesis inhibition prior to the step of administration. In another example, there is provided a method of preventing and/or treating VEGFR2/KDR-related diseases, comprising the step of administering a therapeutically effective amount of the anti-VEGFR2/KDR antibody or antigen-binding fragment thereof to a patient in need of prevention and/or treatment of VEGFR2/KDR-related diseases. The method of preventing and/or treating VEGFR2/KDR-related diseases may further comprise the step of identifying a patient in need of prevention and/or treatment of VEGFR2/KDR-related diseases prior to the administration step.

The pharmaceutical composition may further comprise a pharmaceutically acceptable carrier, wherein the carrier is one conventionally utilized in the formulation of drugs, and may be one or more selected from the group consisting of, but not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oils, and the like. The pharmaceutical composition may further comprise at least one selected from the group consisting of diluents, excipients, lubricants, wetting agents, sweeteners, flavoring agents, emulsifiers, suspending agents, and preservatives, which are conventionally used in the preparation of pharmaceutical compositions.

An effective amount of the pharmaceutical composition, or the antibody or antigen-binding fragment thereof, may be administered orally or parenterally. In the case of parenteral administration, it can be administered by intravenous infusion, subcutaneous infusion, intramuscular infusion, intraperitoneal infusion, endothelial administration, topical administration, intranasal administration, intrapulmonary administration, or rectal administration. Since proteins or peptides are digestible when administered orally, an oral composition may be formulated to coat the active agent or to protect it from degradation in the stomach. Further, the composition may be administered by any device that allows the active agent to be transported to the target cells.

The composition of the present invention is administered in a pharmaceutically effective amount. As used herein, the term "pharmaceutically effective amount" refers to an amount of a pharmaceutical composition for the treatment of a disease that is sufficient with a reasonable benefit/risk ratio applicable to any medical treatment. The effective amount depends on a variety of factors, including the severity of the disease to be treated, the age and sex of the patient, the type of disease, the activity of the agent, the sensitivity to the agent, the time of administration, the route of administration, the rate of secretion, the duration of treatment, co-administration of the agent, and other parameters known in the art. The composition of the present invention may be administered alone or in combination with other therapeutic approaches. In such cases, the composition may be administered in sequence or simultaneously with a conventional therapy. Furthermore, the composition may be administered as a single dose or divided into multiple doses. Taking these factors into full consideration, it is important to administer a minimum amount sufficient to achieve maximum effect without side effects, which can be readily determined by a person skilled in the art. The dosage of the pharmaceutical composition of the present invention is not particularly limited, but varies depending on a variety of factors, including the health status and weight of the patient, the severity of the condition, the type of drug, the route of administration, and the time of administration. The composition may be administered by any generally accepted route of administration in mammals, including rats, mice, cattle, humans, and the like, e.g., orally, rectally, intravenously, intravenously, subcutaneously, intrauterinely, or intracerebrovascularly, in a single dose or multiple doses per day.

The content of the anti-VEGFR2/KDR antibody or antigen-binding fragment thereof in the pharmaceutical composition may be prescribed to vary depending on factors such as the method of formulation, the mode of administration, the age, weight, sex and morbidity of the patient, the food, the time of administration, the interval between administrations, the route of administration, the rate of excretion, and the response sensitivity. For example, the daily dose of the anti-VEGFR2/KDR antibody or antigen-binding fragment thereof may be in the range of, but not limited to, 0.001 to 1000 mg/kg, more particularly 0.01 to 100 mg/kg, more particularly 0.1 to 50 mg/kg, more particularly 0.1 to 20 mg/kg. The daily dose may be formulated as a single unit dose, formulated in suitably divided portions, or formulated in a multi-dose container.

The pharmaceutical composition may be administered in combination with other drugs, such as therapeutic agents for the treatment of other angiogenesis inhibitor-related diseases, and the dosage, method of administration, and type of other drug may be appropriately prescribed depending on the patient's condition.

The pharmaceutical composition may be formulated in the form of a solution, suspension, syrup or emulsion in an oil or aqueous medium, or in the form of an excipient, acid, powder, granule, tablet or capsule, and may further comprise a dispersant or stabilizing agent for formulation.

In particular, a pharmaceutical composition comprising the anti-VEGFR2/KDR antibody or an antigen-binding fragment thereof comprises an antibody or antigen-binding fragment, so it may be formulated as an immunoliposome. Liposomes comprising antibodies can be prepared according to methods well known in the art. The immunoliposome can be prepared by reverse phase evaporation from a lipid composition comprising phosphatidylcholine, cholesterol, and polyethylene glycol-derivatized phosphatidylethanolamine (Korean Laid-open Patent Publication No. 10-2015-0089329). For example, the Fab' fragment of an antibody can be conjugated to liposome via a disulfide-replacement reaction.

In one aspect, the anti-VEGFR2/KDR antibody or antigen-binding fragment thereof binds specifically to VEGFR2/KDR and can therefore be used to determine whether VEGFR2/KDR is activated and/or overproduced. Thus, another example of the present invention provides a pharmaceutical composition for diagnosing a VEGFR2/KDR inhibitor-associated disease, comprising the anti-VEGFR2/KDR antibody or antigen-binding fragment thereof. In another example, there is provided a method of diagnosing or providing information for the diagnosis, comprising: treating a biological sample obtained from a patient with the anti-VEGFR2/KDR antibody or antigen-binding fragment thereof; determining whether an antigen-antibody reaction is present; and, if an antigen-antibody reaction is detected, determining that the patient has symptoms of VEGFR2/KDR activation and/or overproduction, or has a disease associated with activation and/or overproduction of VEGFR2/KDR. The biological sample may be selected from the group consisting of cells, tissues, body fluids, and the like obtained from a patient.

The step of determining the presence of an antigen-antibody reaction can be performed by a variety of methods known in the art. For example, it can be measured by conventional enzymatic reactions, fluorescence, luminescence, and/or radiometric detection, and more specifically, by a method selected from the group consisting of, but not limited to, immunochromatography, immunohistochemistry, enzymeliked immunosorbent assay (ELISA), radioimmunoassay (RIA), enzymeimmunoassay (EIA), fluorescence immunoassay (FIA), luminescence immunoassay (LIA), and western blotting.

By analyzing the final signal intensity by the above immunoassay process, cancer can be diagnosed. That is, if the protein of the marker of the present invention is highly expressed in a biological sample and the signal is stronger than in a normal biological sample (e.g., normal gastric tissue, blood, plasma or serum), cancer is diagnosed.

In another aspect, the present invention relates to a kit for diagnosing cancer comprising the composition for diagnosing cancer. The kit according to the present invention is capable of diagnosing cancer by analyzing a signal represented by the reaction of an antibody with a sample. The signal may comprise, but is not limited to, an enzyme bound to the antibody, such as alkaline phosphatase, β-galactosidase, horseradish peroxidase, luciferase or cytochrome P450, and if an alkaline phosphatase is used as an enzyme, the substrate for the enzyme is a chromogenic reaction substrate such as bromochloroindolyl phosphate (BCIP), nitro blue tetrazolium (NBT), naphthol-AS-B1-phosphate, and enhanced chemifluorescence (ECF), and if horseradish peroxidase is utilized, the substrate is a substrate such as, but not limited to, chloronaphthol, aminoethylcarbazole, diaminobenzidine, D-luciferin, lucigenin (bis-N-methylacridinium nitrate), resorufin benzyl ether, luminol, and Amplex Red reagent (10-acetyl-3,7-dihydroxyphenoxazine), HYR (p-phenylenediamine-HCl and pyrocatechol), TMB (tetramethylbenzidine), ABTS (2,2'-Azine-di[3-ethylbenzthiazoline sulfonate]), o-phenylenediamine (OPD), naphthol/pyronine, glucose oxidase, t-NBT (nitroblue tetrazolium) or m-PMS (phenzaine methosulfate).

Further, the kit according to the present invention may comprise a label that generates a detectable signal, wherein the label may include, but is not limited to, a chemical substance (e.g., biotin), an enzyme (alkaline phosphatase, β-galactosidase, horseradish peroxidase, and cytochrome P450), a radioactive substance (e.g., C14, I125, P32 and S35), a fluorescent substance (e.g., fluorescein), a luminescent substance, a chemiluminescent substance and a fluorescence resonance energy transfer (FRET).

Measurement of enzyme activity or signaling used for the diagnosis of angiogenesis can be performed according to a variety of methods known in the art.

The patient to whom the pharmaceutical composition is administered or diagnosed may be a mammal, including a primate including a human and a monkey, and a rodent including a mouse and a rat.

The VEGFR2/KDR-associated disease may be cancer; cancer metastasis; eye diseases, such as retinopathy of prematurity, macular degeneration (e.g., age-related macular degeneration), diabetic retinopathy, angiogenic glaucoma; asthma; rheumatoid arthritis; psoriasis; inflammatory diseases, such as pneumonia or chronic inflammation; cardiovascular diseases, such as hypertension and atherosclerosis; or sepsis. The cancer may overexpress VEGFR2/KDR and may be a solid or hematologic cancer, and may be at least one selected from the group consisting of, but is not limited to, squamous cell carcinoma, small cell lung cancer, non-small cell lung cancer, adenocarcinoma of the lung, squamous cell carcinoma of the lung, peritoneal cancer, skin cancer, cutaneous or intraocular melanoma, rectal cancer, anorectal cancer, esophageal cancer, small bowel cancer, endocrine gland cancer, parathyroid cancer, adrenal cancer, soft tissue sarcoma, urethral cancer, chronic or acute leukemia, lymphocytic lymphoma, hepatocellular carcinoma, gastrointestinal cancer, pancreatic cancer, glioblastoma, cervical cancer, ovarian cancer, liver cancer, bladder cancer, liver tumor, breast cancer, colon cancer, colorectal cancer, endometrial or uterine cancer, salivary gland cancer, kidney cancer, liver cancer, prostate cancer, vulva cancer, thyroid cancer, liver cancer, head and neck cancer, brain cancer, and osteosarcoma. The cancer may be a primary cancer or a metastatic cancer.

Furthermore, the antibody of the present invention can be used in combination with other antibodies or biologically active agents or substances for various purposes. The present invention relates in this respect to a composition for co-administration with other therapeutic agents for angiogenic diseases, comprising the antibody or antigen-binding fragment thereof.

Other angiogenic disease treatment agents may include anti-angiogenic drugs, anti-inflammatory drugs, and/or anti-cancer drugs. These can overcome each other's resistance and enhance efficacy.

When co-administered with another angiogenesis therapeutic agent in a composition according to the present invention, the anti-VEGFR2/KDR antibody or antigen-binding fragment thereof and the other angiogenesis therapeutic agent may be administered sequentially or simultaneously. For example, an anti-angiogenic drug, an anti-inflammatory drug, and/or an anti-cancer drug is administered to a subject followed by administration to the subject of a composition comprising an anti-VEGFR2/KDR antibody or an antigen-binding fragment thereof as an active ingredient, or the composition is administered to the subject followed by administration to the subject of an anti-angiogenic drug, an anti-inflammatory drug, and/or an anti-cancer drug. In some cases, the composition may be administered to the subject simultaneously with an anti-angiogenic drug, an anti-inflammatory drug, and/or an anti-cancer drug.

### Examples

The present invention is described in more detail by providing the following examples. These examples are intended solely to illustrate the present invention, and it would be apparent to one of ordinary skill in the art that the scope of the present invention is not to be construed as limited by these examples.

### Example 1. CDR grafting

The CDR sequence of 6A6 was identified by Kabat numbering and IMGT program, and it was cloned into the Human germline VH3/VK1.

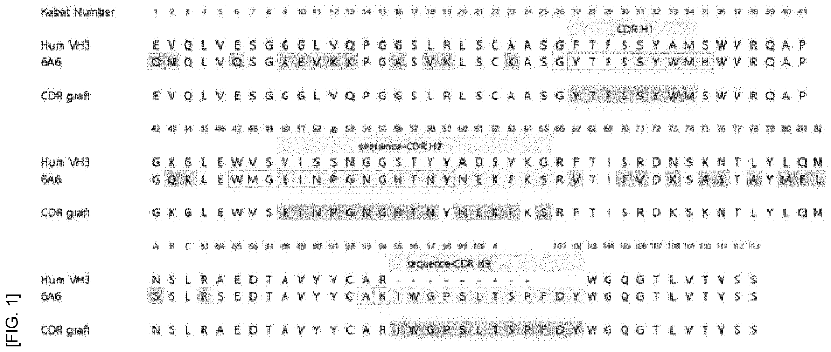

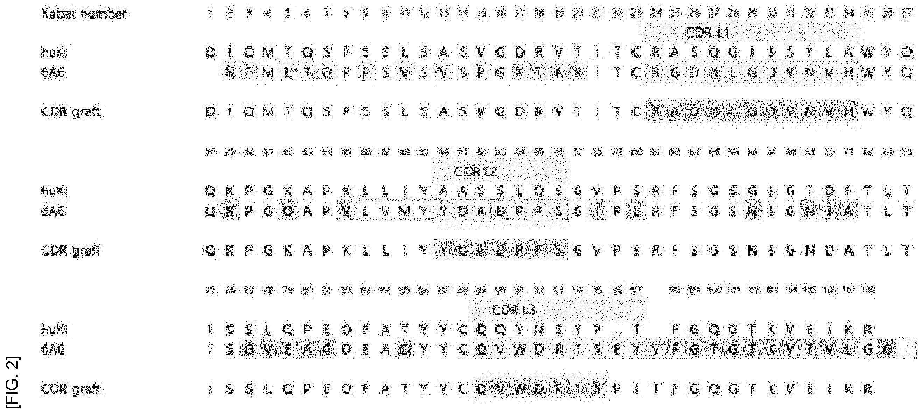

FIG. 1 shows the sequence of the heavy chain CDR, and FIG. 2 shows the sequence of the light chain CDR.

### Example 2. Producing and screening variants for increased affinity

Optimization was performed to improve the affinity of the CDR grafted antibody. Using the soft-randomization method, which randomizes the original DNA sequence of CDR3 of light and heavy chain with 70% conservation, a primer introducing random variations in light chain CDR3 and heavy chain CDR3 was prepared. PCR using the primer yielded DNA fragments coding the modified light and heavy chain variable regions. These DNA fragments were substituted with the light chain variable region of the scFv phage phagemid and the heavy chain variable region of the scFv phage phagemid, respectively, to create a light chain CDR3 mutant scFv phage library and a heavy chain CDR3 mutant scFv phage DNA library, respectively.

The variant scFv phage DNA library was transformed into E. coli strain XL-1 Blue using electroporation method after phenol-chloroform purification. After analyzing the transformation efficiency and confirming the diversity via DNA sequencing, phage expression was induced by incubation at 500 ml scale, and a library of CDR3 variant scFv phages of light and heavy chains was prepared by PEG-precipitation method.

Biopanning was performed using each variant scFv phage library. Antigens (KDR1 to 3 domain-Fc) were added to 96-well immunization plates in an amount of 100 ml at a density of 2 mg/ml and incubated at 4°C overnight. The next day, the antigen-coated plates were washed three times with PBST (0.1% tween20), followed by addition of 200 ml of 2% BSA blocking buffer, and reacted for 2 hours at room temperature. 50 ml of XL1-Blue stock was added to 2 ml of 2x YT-TET (tetracycline 10 µg/ml) growth medium and incubated at 37°C at 200 rpm for 2 hours, then 13 ml of XL1-Blue stock was further added to increase an OD₆₀₀ to 0.5. After 2 hours of blocking, the wells were washed three times with 1X PBST (0.1% tween20). The variant library and 4% BSA were mixed in the same amount, and 200 ml of the mixture was added to each washed well, and the wells were rocked for 30 minutes at room temperature, and reacted for 2 hours. At the end of the phage library reaction, the supernatant was discarded and each well was washed five times with 0.1% PBST and five times with PBS. 100 ml of 100 mM trimethylamine (TEA) was added to each well and shaken for 10 minutes at room temperature. After 10 minutes, 50 ml of 1M Tris (pH 7.5) was added to each well and mixed. The supernatant was added to 10 ml of XL1-blue with an OD₆₀₀ of 0.5 and infected at 37°C for 30 minutes. At the end of the infection, 100 ml was used as an output titer and the rest was centrifuged at 6,000 rpm for 10 minutes. The supernatant was discarded and the precipitate was spread on a large square plate (CM 34 µg/ml + 1% Glucose) and incubated overnight at 37°C. The 100 ml reserved for the output titer was diluted to concentrations of 1/10, 1/100, and 1/1000 and spread on CM plates and incubated overnight at 37°C. The next day, the colonies grown on the square plates were scraped off using a loop after adding 50 ml of 2x YT medium and centrifuged at 6000 rpm for 10 min, discarding the supernatant, and a primary panning stock was made to the precipitate, 100 ml of 2x YT culture medium (growth media: CM 34 µg/ml + 1% Glucose) was added to a 500ml trifold flask, and then the cells were added to increase an OD₆₀₀ to 0.2, and incubated until an OD₆₀₀ grows to 0.5 at 200 rpm and 37°C. After the cells were incubated until an OD₆₀₀ grows to 0.5, a helper phage (M13KO7 mutant) was added to 20 times the amount of the cells. After adding the helper phage, the cells were infected at 37°C for 30 min and centrifuged at 6000 rpm for 10 min. The supernatant was discarded and the cells were replaced with 100 ml of 2xYT medium (CM 34 µg/ml + Kan. 70 µg/ml + 1 mM IPTG + 5 mM MgCl₂) and incubated overnight at 200 rpm at 30°C. The next day, the grown cells were centrifuged at 7000 rpm for 10 min, and centrifuged once more in the same way. The collected supernatant was precipitated for 1 hour on ice with 1/5 of the supernatant (v/v) 20% PEG/2.5 m NaCl. After precipitation, the supernatant was centrifuged at 9000 rpm for 1 hour. The supernatant was discarded and the precipitate was dissolved with 5 ml of PBS, filtered through a 0.45 mm filter, and stored at 4°C to be used in the next round of panning. The process was repeated three to four times and the antibody binding to the antigen was confirmed by ELISA.

The screening process then measured the dissociation rate constant K_{dis} of scFv as a quantitative measure of its ability to maintain binding.

### Example 2. Off-rate screening of antibodies with high affinity for an antigen

The binding of the selected antibodies to the antigen was measured using Octet (Fortebio). For this purpose, the KDR1 to 3 domains were immobilized on a biosensor, the candidate antibodies expressed as scFv were added and bound, and the dissociation rate constants were measured. Tables 2 and 3 show the amino acid sequences for the five selected optimized clones and Table 1 shows the results of the dissociation rate constant measurements. The CDR sequences indicated below are those analyzed through the IMGT database.

**[Table 1] Dissociation rate constants for antibodies that specifically bind to an antigen**

| clone | K_{dis} (1/s) |
|---|---|
| 6A6 | 2.66E-04 |
| CDR grafted | 2.97E-04 |
| LH-E7 | 1.11E-,04 |
| LHL-G1 | 2.39E-04 |
| D4 | 1.65E-04 |
| A5 | 1.87E-04 |
| D3 | 1.50E-04 |

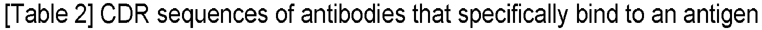

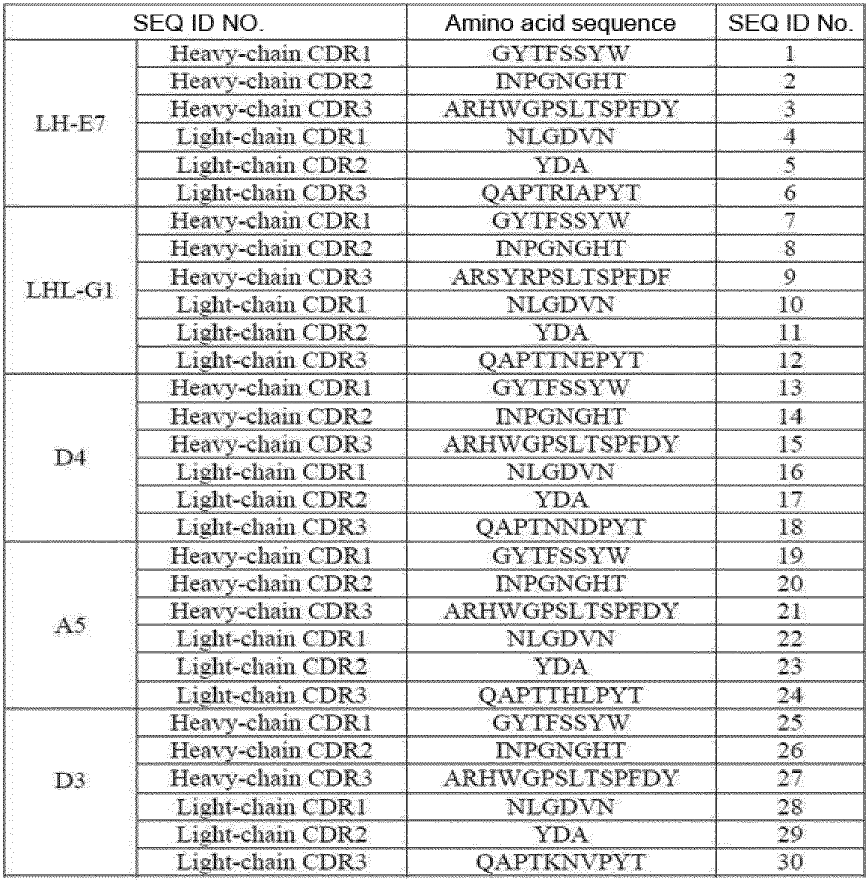

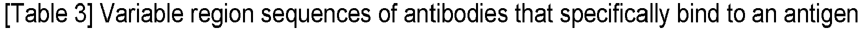

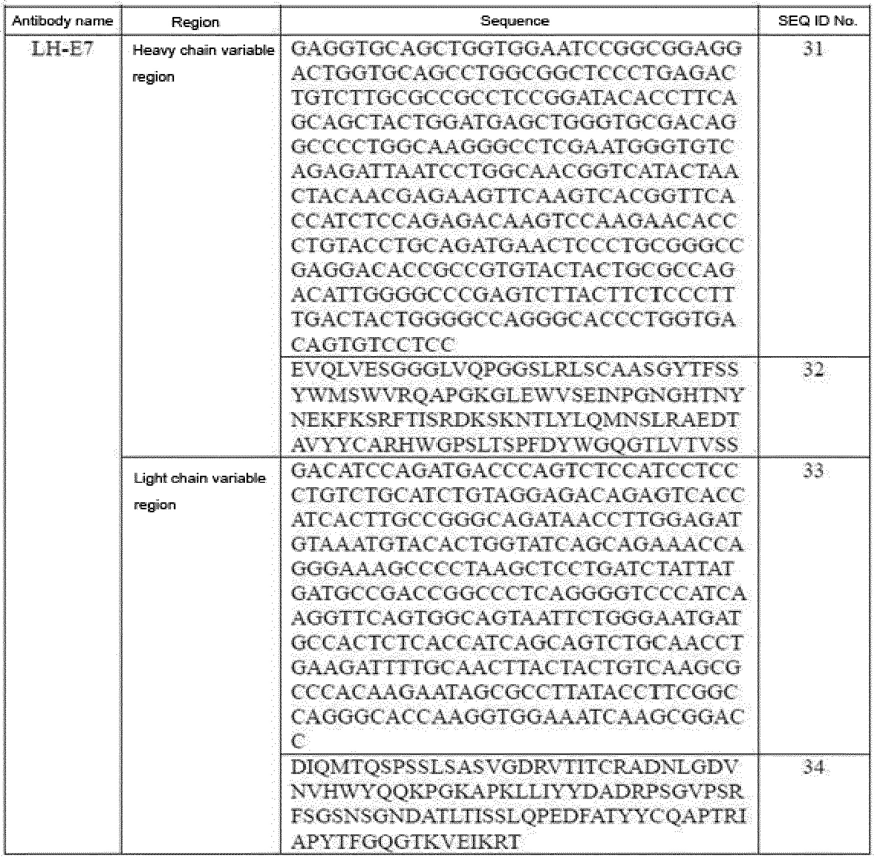

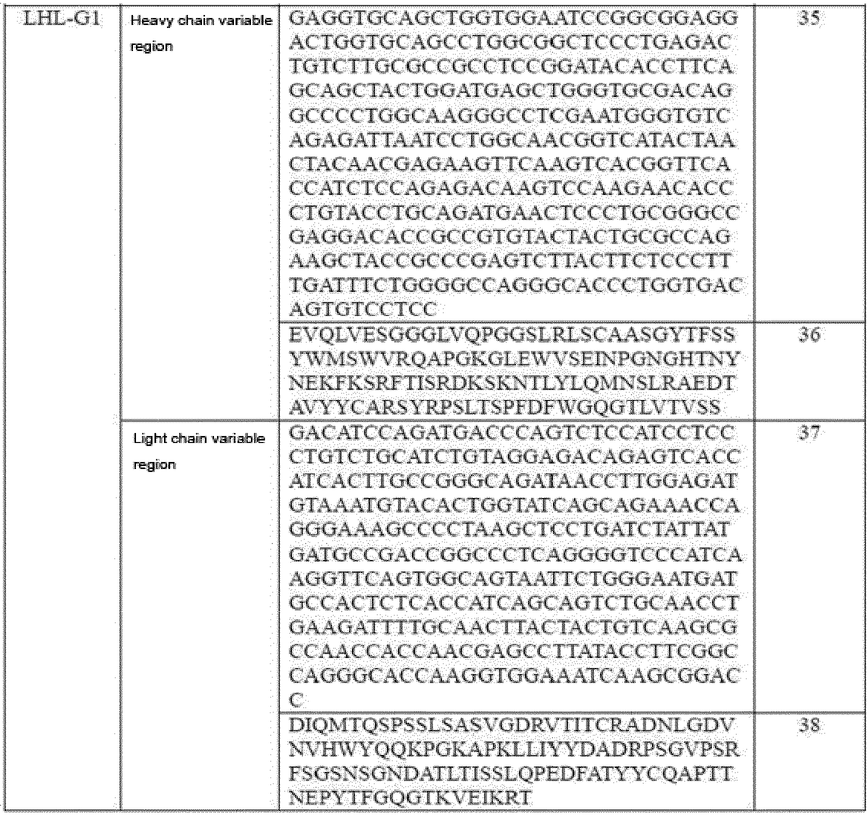

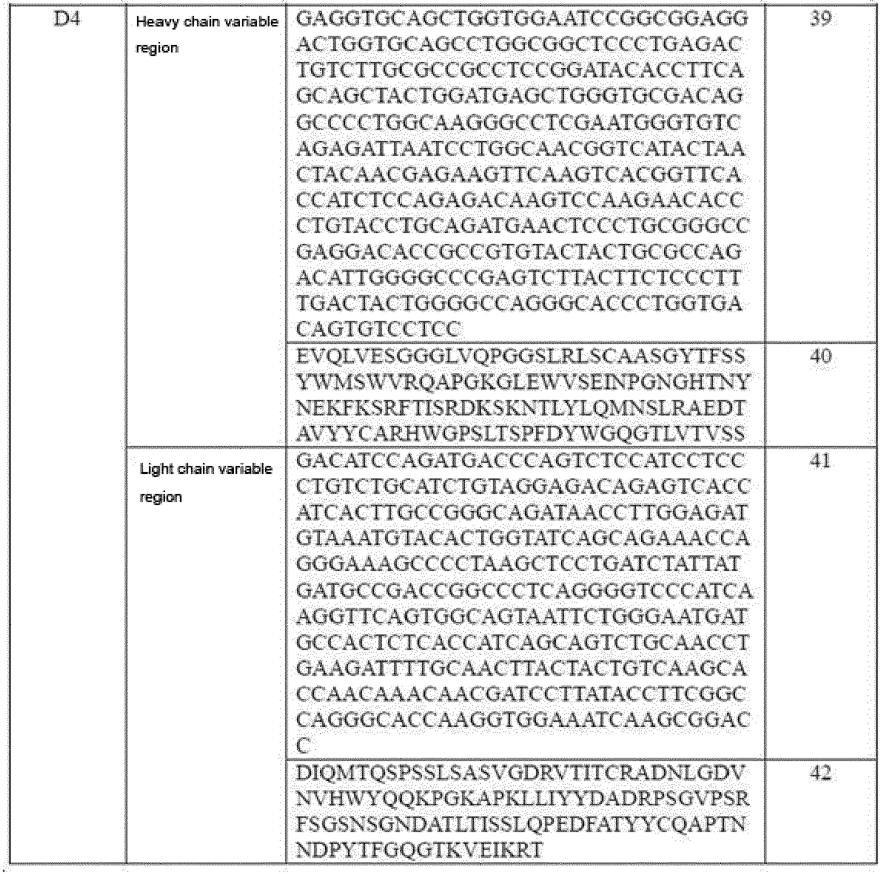

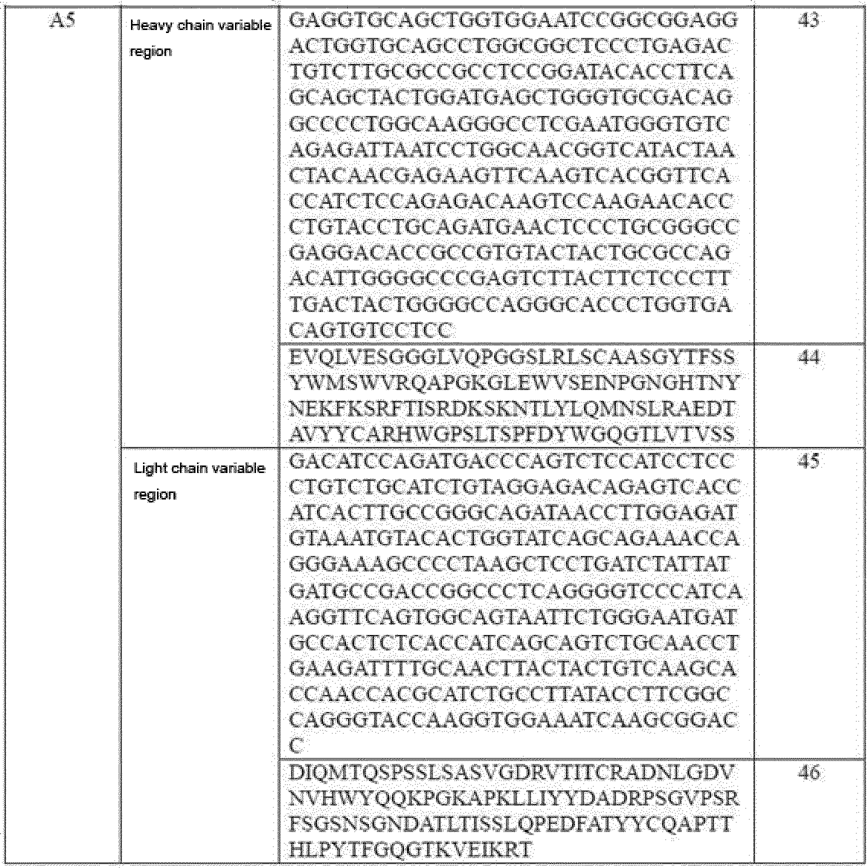

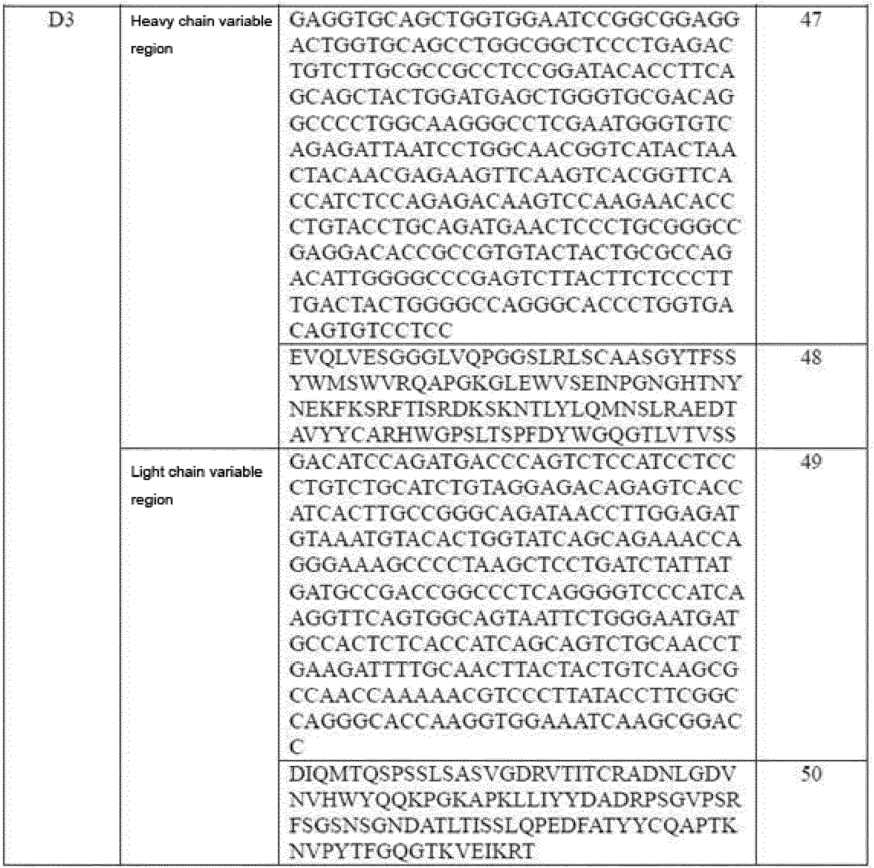

### Example 3. Antibody expression

Conversion of selected scFv phages to IgG form was performed using molecular biology techniques. Phagemid was extracted from selected E. Coli clones and the variable region was amplified using PCR. The amplified heavy chain variable region was inserted into an expression vector (Invivogen, pfusess-hchg1) containing the heavy chain invariant region, and the amplified light chain variable region was inserted into an expression vector (Invivogen, pfuse2ss-hclk) containing the light chain invariant region to complete the DNA cloning of the IgG form.

Transient expression of IgG was performed using the Expi293F expression system kit (Thermo Fisher Scientific, US). Expi293 cells contained in the kit were floating cultured on a 125 rpm orbital shaker at 37°C under 5% CO2 environment using dedicated medium. The cells were subcultured every 3 days to reach 3 × 10⁵ cells/ml, and the cell number was adjusted to 3 × 10⁶ cells/ml for expression vector transfection. Gene transfection was performed by using the proprietary reagent Expifectamine, and lipid-DNA complexes containing 1 mg of expression vector DNA and 2.7 µl of Expifectamine per ml of cell suspension were prepared and added to the cell suspension, and expression was induced 16-18 hours after transfection by adding enhancer 1/2. The cells were then incubated under the same conditions for 3-4 days and centrifuged to collect the IgG-containing supernatant.

### Example 4. Purifying antibodies

The obtained supernatant was injected onto a Protein A column (GE Healthcare) to purify IgG by affinity chromatography. The column was equilibrated with 20 mM Tris-HCl, 50 mM NaCl, and 5 mM EDTA (pH 7.0) and the supernatant was injected, washed with a solution of 50 mM Tris-HCl, 500 mM NaCl, 5 mM EDTA, and 0.2% polysorbate 20 (pH 7.0), eluted with 50 mM NaCl, and 0.1 M glycine-HCl (pH 3.5), and neutralized with 1 M Tris. In the eluted proteins, the solvent was replaced with PBS via dialysis process using MWCO 10,000 spectra/por dialysis membrane (Spectrum Labs, US). It was then concentrated to the required concentration using Vivaspin (Satorius, DE) and dispensed, and then stored at -80°C.

### Example 5. Analyzing the binding specificity of antibodies

The binding of the selected antibodies to the KDR1 to 3 domain was measured using the Octet system (Fortebio Inc. US). For this purpose, anti-VEGFR2/KDR antibodies were immobilized on the biosensor, and the concentration-dependent binding kinetics of human KDR1 to 3 were measured to calculate the binding rate constant (kₐ), dissociation rate constant (k_{dis}), and binding constant (K_{D}) (Table 4).

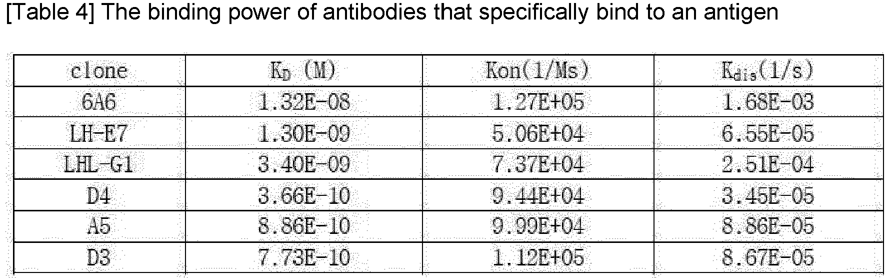

### Example 6. Determining neutralizing ability of antibodies (HUVEC viability assay)

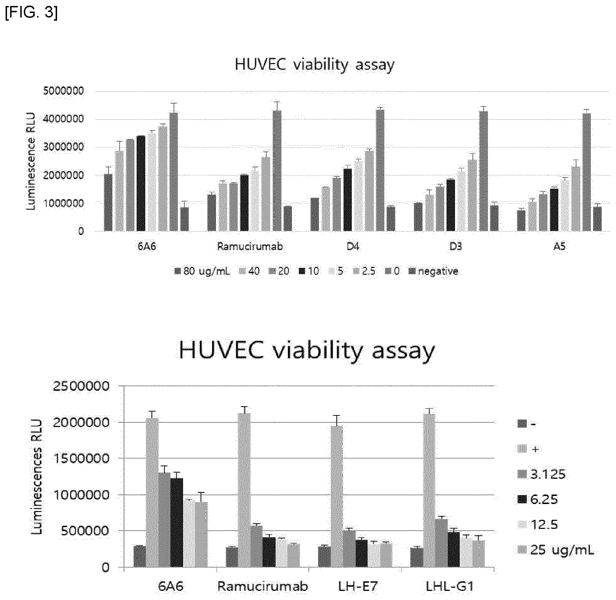

The following experiments were performed to measure the biological activity of the antibody. HUVEC was performed by using cells purchased from Lonza (cat# C2519A) and cultured in 2% gelatin coated plates using VascuLife VEGF-Mv medium complete kit (Lifeline cell technology, cat# LCT-LL-0005) medium. Culture is performed in an incubator with 5% Co2 at 37°C. Cells within passage 10 are used for the assay, which was performed using 1×10⁴ cells per well in 96-well plates with M199 medium (Gibco, cat#11043-023) containing 0.5% heat inactivated FBS. The assay was performed by first treating the assay plate with antibodies diluted at different concentrations, then adding 1×10⁴ cells per well and incubating the cells in the incubator for 30 minutes. After 30 minutes of incubation, VEGF165 (R&D, cat#293-VE) was treated at a concentration of 30 ng/mL per well. After incubation in an incubator with CO₂ at 37°C for 3 days, viability was measured. Viability was determined by quantifying ATP in living cells using the CellTier Glo-Luminescent Cell viability Assay Kit (cat no. G7571) from promega (FIG. 3). As shown in FIG. 3, the selected antibodies inhibited the effects of VEGF165 in a concentration-dependent manner.

### Example 8. Analyzing the effect of the antibody in inhibiting VEGF165/VEGFR2 signaling (p-KDR assay)

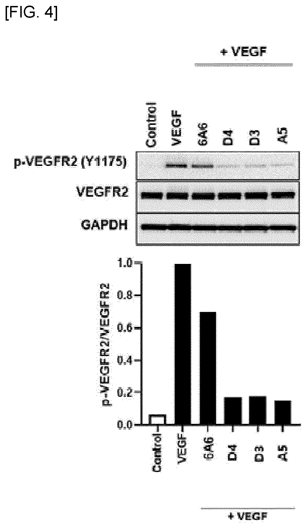

HUVEC cells were used to analyze the inhibitory effect of VEGF/VEGFR2 signaling, and HUVECs were purchased from Lonza (cat# C2519A). Cells were cultured using VascuLife VEGF-Mv medium complete kit (Lifeline cell technology, cat# LCT-LL-0005) medium, and 8×10⁵ of the cells were added per well in 6-well plates, and grown overnight in an incubator with carbon dioxide at 37°C. The antibody was treated with antibody at a concentration of 10 ug/mL for 20 minutes, followed by VEGF165 (R&D, cat#293-VE) for 10 minutes. Wells without antibody and containing only VEGF165 protein were included as a reference for the signaling inhibition assay. After each drug treatment, HUVECs were washed twice with PBS and reacted with lysis buffer (Thermo Scientific, RIPA buffer & phosphatase inhibitors) to obtain cell extracts and incubated for 20 min on ice. After centrifugation at 13000 rpm at 4°C, only the supernatant was obtained. After quantification using the BCA protein quantification method, the lysate was loaded onto a 10% SDS-PAGE gel and electrophoresed. After transferring using a nitrocellulose membrane (Bio-Rad), 5% skim milk was diluted in 0.1% Tween20 TBST buffer and blocked for 1 hour at room temperature. Then, primary antibody (p-VEGFR2(Y1175) ; Cell signaling) was diluted to a concentration of 1:1000 and shaken overnight at 4°C, and peroxidase-conjugate secondary antibody (Santa cruz) was diluted to a concentration of 1:5000 and reacted for 1 hour at room temperature. The bands of the target proteins were observed by ECL solution (Pierce) reaction. As the results, FIG. 4 showed that the phosphorylation by VEGF165 was more effectively inhibited in D4, D3 and A4 than that of 6A6.

### Industrial availability

The present invention developed an antibody with improved affinity over existing antibodies targeting VEGFR2/KDR, which may provide more efficacious combined or single treatment therapy for tumors than existing therapies. The anti-VEGFR2/KDR antibody or antigen-binding fragment thereof according to the present invention exhibits enhanced binding to VEGFR2/KDR and may be useful in the prevention or treatment of a targeted cancer/tumor or angiogenesis inhibitor-related diseases.

While the foregoing has described in detail certain aspects of the present invention, it would be apparent to one of ordinary skill in the art that these specific descriptions are merely preferred embodiments and are not intended to limit the scope of the present invention. Accordingly, the substantial scope of the present invention is defined by the appended claims and their equivalents.

### Sequence list

Attached electronically.

## Claims

1. An antibody or antigen-binding fragment thereof that binds to VEGFR2/KDR, comprising:
a heavy chain CDR1 comprising a sequence selected from the group consisting of sequence ID Nos: 1, 7, 13, 19 and 25,
a heavy chain CDR2 comprising a sequence selected from the group consisting of sequence ID Nos: 2, 8, 14, 20 and 26; and
a heavy chain variable region comprising a heavy chain CDR3 comprising a sequence selected from the group consisting of sequence ID Nos: 3, 9, 15, 21 and 27; and
a light chain CDR1 comprising a sequence selected from the group consisting of sequence ID Nos: 4, 10, 16, 22 and 28,
a light chain CDR2 comprising a sequence selected from the group consisting of sequence ID Nos: 5, 11, 17, 23 and 29; and
a light chain variable region comprising a light chain CDR3 comprising a sequence selected from the group consisting of sequence ID Nos: 6, 12, 18, 24 and 30.

2. The antibody or antigen-binding fragment thereof according to claim 1, comprising:
a heavy chain variable region comprising a heavy chain CDR1 of SEQ ID NO: 1, a heavy chain CDR2 of SEQ ID NO: 2, and a heavy chain CDR3 of SEQ ID NO: 3; and a light chain variable region comprising a light chain CDR1 of SEQ ID NO: 4, a light chain CDR2 of SEQ ID NO: 5, and a light chain CDR3 of SEQ ID NO: 6,
a heavy chain variable region comprising a heavy chain CDR1 of SEQ ID NO: 7, a heavy chain CDR2 of SEQ ID NO: 8, and a heavy chain CDR3 of SEQ ID NO: 9; and a light chain variable region comprising a light chain CDR1 of SEQ ID NO: 10, a light chain CDR2 of SEQ ID NO: 11, and a light chain CDR3 of SEQ ID NO: 12,
a heavy chain variable region comprising a heavy chain CDR1 of SEQ ID NO: 13, a heavy chain CDR2 of SEQ ID NO: 14, and a heavy chain CDR3 of SEQ ID NO: 15; and a light chain variable region comprising a light chain CDR1 of SEQ ID NO: 16, a light chain CDR2 of SEQ ID NO: 17, and a light chain CDR3 of SEQ ID NO: 18,
a heavy chain variable region comprising a heavy chain CDR1 of SEQ ID NO: 19, a heavy chain CDR2 of SEQ ID NO: 20, and a heavy chain CDR3 of SEQ ID NO: 21; and a light chain variable region comprising a light chain CDR1 of SEQ ID NO: 22, a light chain CDR2 of SEQ ID NO: 23, and a light chain CDR3 of SEQ ID NO: 24, or
a heavy chain variable region comprising a heavy chain CDR1 of SEQ ID NO: 25, a heavy chain CDR2 of SEQ ID NO: 26, and a heavy chain CDR3 of SEQ ID NO: 27; and a light chain variable region comprising a light chain CDR1 of SEQ ID NO: 28, a light chain CDR2 of SEQ ID NO: 29, and a light chain CDR3 of SEQ ID NO: 30.

3. The antibody or antigen-binding fragment thereof according to claim 1, comprising a heavy chain variable region selected from the group consisting of SEQ ID Nos: 32, 36, 40, 44 and 48.

4. The antibody or antigen-binding fragment thereof according to claim 1, comprising a light chain variable region selected from the group consisting of SEQ ID Nos: 34, 38, 42, 46 and 50.

5. The antibody or antigen-binding fragment thereof that binds to VEGFR2/KDR according to claim 1, comprising a heavy chain variable region comprising a sequence having at least 80% identity to a sequence selected from the group consisting of SEQ ID Nos: 32, 36, 40, 44 and 48.

6. The antibody or antigen-binding fragment thereof that binds to VEGFR2/KDR according to claim 1, comprising a light chain variable region comprising a sequence having at least 80% identity to a sequence selected from the group consisting of SEQ ID Nos: 34, 38, 42, 46 and 50.

7. The antibody or antigen-binding fragment thereof that binds to VEGFR2/KDR according to claim 1, comprising a heavy chain variable region comprising a sequence having at least 80% identity to a sequence selected from the group consisting of SEQ ID Nos: 32, 36, 40, 44 and 48, and a light chain variable region comprising a sequence having at least 80% identity to a sequence selected from the group consisting of SEQ ID Nos: 34, 38, 42, 46 and 50.

8. A nucleic acid encoding the antibody or antigen-binding fragment thereof according to any one of claims 1 to 7.

9. An expression vector comprising the nucleic acid according to claim 8.

10. A transfected cell, comprising the expression vector according to claim 9.

11. A method of making an antibody or antigen-binding fragment thereof that binds to VEGFR2/KDR, comprising the following steps:
(a) culturing the cells of claim 10; and
(b) recovering the antibody or antigen-binding fragment thereof from the cultured cells.

12. A composition for the prevention or treatment of angiogenic diseases, comprising the antibody or antigen-binding fragment thereof according to any one of claims 1 to 7 as an active ingredient.

13. A composition for diagnosing angiogenic diseases, comprising the antibody or antigen-binding fragment thereof according to any one of claims 1 to 7 as an active ingredient.

14. A composition for the prevention or treatment of tumor or cancer, comprising the antibody or antigen-binding fragment thereof according to any one of claims 1 to 7 as an active ingredient.

15. A composition for co-administration with other therapeutic agents, comprising the antibody or antigen-binding fragment thereof according to any one of claims 1 to 7.
